# EUROPEAN PATENT APPLICATION

(11) **EP 4 410 771 A1**
(43) Date of publication of application: **07.08.2024**
(21) Application number: 23154927.0
(22) Date of filing: 03.02.2023
(51) Int. Cl.: C07C 67/03, C07C 69/26, C07C 69/30, C11B 1/02, B01J 21/10, B01J 23/04, B01J 27/16, C11C 3/10

(54) **ORGANOTIN FREE CATALYSTS FOR TRANSESTERIFICATION WITH MONO- AND POLYFUNCTIONAL ALCOHOLS**

(71) Applicant: BASF SE, 67056 Ludwigshafen am Rhein (DE)
(72) Inventor: Roessler, Harald, 40589 Duesseldorf (DE); Mahnke, Eike Ulf, 40589 Düsseldorf-Holthausen (DE); Kopka, Joerg, 40589 Duesseldorf (DE); Krueppel, Heinz-Josef, 40589 Duesseldorf (DE); Muckenschnabel, Christian, 40589 Duesseldorf (DE); Scholinakis, Konstantinos, 40589 Düsseldorf-Holthausen (DE)
(74) Representative: BASF IP Association

(57) **Abstract**

The invention is related to a transesterification process comprising treating an alkyl ester of a carboxylic acid (a) of formula 1a or 1b

R¹-C(O)O-R² (formula 1a)

R²-(O)C-O-R³-O-C(O)-R² (formula 1b)

wherein R¹ and R³ is the saturated or unsaturated, branched or linear aliphatic or aromatic or alkoxylated residue and R² is selected from the group consisting of methyl, ethyl, propyl, isopropyl, n-butyl, and 2,2-butyl,
with a monofunctional alcohol (b1) of formula 2,

R⁴-OH (formula 2),

wherein R⁴ is a linear or branched, saturated or unsaturated alkyl or a polyfunctional alcohol (b2) selected from glycerol, 1, 3-propylenglycol, 1,2-propylenglycol, ethylenglycol, 1,2-butandiol, 1,4-butandiol, 2,3-butandiol
in the presence of the catalysts lithiumhydroxide and magnesiumoxide (MgO) and optionally sodium hypophosphite. The use of the specific catalyst composition enables an energy-saving more efficient process than standard prior art transesterification processes.

## Description

Process technology today aims at environmentally friendly catalysts and environmentally friendly processes for the chemical synthesis of raw materials especially for use in cosmetic and pharmaceutical products. Many processes established with conventional catalysts are currently being converted to more environmentally friendly catalysts. Thereby it is of major concern that the products that originate from the converted processes will meet the specification of the products resulting from the original processes and that as few process control changes as possible are necessary.

Organotin catalysts such as dibutyltin diacetate or tetrabutyldiacetoxy distannoxane are well established transesterification catalysts in the chemical industry. Nevertheless, already in 2003 the EU banned tributyltin in marine antifouling coatings followed by a ban of tributyltin in consumer products in 2010 in Germany because of its damaging effects on genetic and fertility. Hence the risk of organotin residues in cosmetic products is an increasing threat for customer and consumer acceptance. In addition organotin catalysts are difficult to remove from the esterified product, which results in costly and energy- intensive purification processes. Common catalysts that are active above 180 °C, such as organotin compounds, must be separated from the raw product by destruction of the carbon-tin bond by oxidation to tinoxide, aqueous precipitation and filtration with filter aids.

The European patent application EP1858480 describes esters of 2-propylheptanol with linear or branched, saturated or unsaturated C4-C36 carboxylic acids or C4-C36 dicarboxylic acids, which are used in cosmetic and / or pharmaceutical preparations as emollients. These esters are prepared by the reaction of 2-propylheptanol with the corresponding carboxylic acids at temperatures of 100 to 300 ° C using sodium methylate and tetraalkyl titanate as catalysts. Also alkyltitanates are unwanted by-products in cosmetic and personal care products. Furtherone the use of alkyltitanate results in unwanted side products by reaction with the alkyl ester educt. This catalyst affords additional hydrolysis steps in order to de removed from the reaction product after transesterification. In addition some of these catalysts require very high temperatures to develop their catalytic effect. High energy consumption is thus another drawback of this process which should be avoided when developing new manufacturing processes.

In US patent US20100197955 a process for preparing butanediol dimethacrylates is disclosed, which comprises the transesterification of an ester of methacrylic acid with butanediol in the presence of catalysts containing lithium hydroxide and calcium oxide. The use of this catalyst combination for preparing alkyl fatty acid esters has not resulted in a sufficiently stable and clear esterification product for fatty acid esters because of a slurry developed during storage.

Therefore the aim of the invention is the provision of a tin and titanate -free catalyst that is selective and highly active under similar or even milder conditions than organotin catalysts. In particular, a lower reaction temperature (at least ≤ 190 °C) and a reaction start at even lower temperatures should be sought. In addition, a simple separation and removal of the new catalyst (by combination of precipitation and filtration) is desirable. The resulting reaction product should have the requested specification in view of catalyst removal and sufficient stability in comparison with the one prepared with the former catalysts. The implementation in the production operation should be possible with little effort. Besides using a catalyst with a better toxicological profile also the decrease of energy consumption should as well contribute to achieve a more environmentally friendly production process.

### Description of the Invention

Surprisingly a transesterification process comprising
treating an alkyl ester of a carboxylic acid (a) of formula 1a or 1b

   R¹-C(O) O-R² (formula 1a)

   R²-(O)C - O-R³-O-C(O)-R² (formula 1b)
wherein R¹ and R³ is the saturated or unsaturated, branched or linear aliphatic or aromatic residue and R² is selected from the group consisting of methyl, ethyl, propyl, isopropyl, n-butyl, and 2,2-butyl,
with a monofunctional alcohol (b1) of formula 2,

   R⁴-OH (formula 2),
wherein R⁴ is a linear or branched, saturated or unsaturated or alkoxylated alkyl-, alkenyl- or alkoxy residue or
a polyfunctional alcohol (b2) selected from glycerol, 1, 3-propylenglycol, 1,2-propylenglycol, ethylenglycol, 1,2-butandiol, 1,4-butandiol, 2,3-butandiol
in the presence of the catalysts lithiumhydroxide and magnesiumoxide (MgO) and optionally sodium hypophosphite resulted in a clear and stable reaction product.

Surprisingly the catalyst combination of lithium hydroxide and magnesiumoxide reacts already far below 100°C, whereas the transesterification with other catalysts, especially tin containing catalysts, starts at about 150°C. A higher yield already at lower temperature using lithium hydroxide and magnesiumoxide is advantageous to run an energy saving process. Usually the transesterification stagnates at a yield of approximately 90 % yield, which is then reached earlier with less energy consumption. Despite the lower reaction temperature, which puts less stress on the product and is more energy-saving, the reaction time is comparable to the one of the former standard process.

In addition further processing of the ester is simplified as the effort to remove the catalysts and phosphor as salt of the phosphoric acid is much lower. The salts can already be excellently separated during filtration, so that a simple filtration step may be sufficient to purify the product.

### Component a) - alkyl ester of a carboxylic acid

Moiety R¹ of the alkylester of the mono carboxylic acid according to formula 1a is a linear, branched, saturated or unsaturated C1 -C 36 alkyl, preferably a linear, branched, saturated or unsaturated alkyl from C4 to C30, particularly C6 to C24, more particularly C6 to C22, still more particularly C6 to C18, most particularly C8 to C18, preferably C8 to C16, more preferably C8 to C12, and still more preferably C6 to C10 alkyl.

Moiety R² of the alkyl esters of formula 1a and formula 1b is selected from the group consisting of methyl, ethyl, propyl, isopropyl, n-butyl, and 2,2-butyl, preferably from the group consisting of methyl, ethyl and isopropyl and most preferably R² is methyl.

The moiety R³ of the alkylester with dicarboxylic acids according to formula 1b is a linear, branched, saturated or unsaturated C2 to C54, particularly C4 to C36, more particularly C6 to C22, still more particularly C6 to C18, most particularly C8 to C18, preferably C8 to C16, more preferably C8 to C12, and still more preferably C6 to C10 alkyl. R³ could as well result from dimer fatty acids, polycarboxylic acids which are obtained by polymerization of unsaturated fatty acids, mainly oleic acid or tall oil fatty acid so that R³ contains an alkyl moiety from a C36 dicarboxylic acid.

Preferred dicarbonic acid ester are ester of phthalic acid, terephthalic acid, sebacic acid, azelaic acid, adipic acid and dodecanoic diacid.

### Component b) - the alcohol

The moiety R⁴ of formula 2 is a linear or branched, saturated or unsaturated or alkoxylated alkyl-, alkenyl- or alkoxy residue, preferably a linear or branched, saturated, unsaturated C2 to C36 alkyl, more preferably a linear, branched, saturated or unsaturated C3 to C24 alkyl, most preferably a linear or branched, saturated C4 to C12 alkyl.

Specifically the monofunctional **alcohol** (b1) is 2-propylheptanol and the polyfunctional alcohol (b2) is glycerol.

Esters of glycerol such as mono-, di and triesters of glycerol or esters of 2-propyl-heptanol prepared according to the inventive process are particularly suitable for cosmetic formulations. These esters may be incorporated particularly well in various formulations, including liquid mixtures that may be used as oil components or consistency factors according to chain length, branching and number of double bonds of these esters.

Ester of 2-propylheptanol with linear or branched, saturated or unsaturated C5-C36 carboxylic acids or C4-C36 dicarboxylic acids, more preferably ester of 2-propylheptanol with linear or branched, saturated or unsaturated C5-C18 carboxylic acids or C4-C18 dicarboxylic acids, most preferably ester of 2-propylheptanol with linear or branched, saturated or unsaturated C6-C12 carboxylic acids such as 2-propylheptyl-n-butanoic acid ester, 2-propylheptyl-i-butanoic acid ester, 2-propylheptyl-n-pentanoic acid ester, 2-propylheptyl-i-pentanoic acid ester, 2-propylheptyl-n-hexanoic acid ester, 2-propylheptyl-i-hexanoic acid ester, 2-propylheptyl-n-heptanoic acid ester, 2-propylheptyl-i-heptanoic acid ester, 2-propylheptyl-i-octanoic acid ester, 2-propylheptyl-n-nonanoic acid ester, 2-propylheptyl-i-nonanoic acid ester, 2-propylheptyl-n-decanoic acid ester, 2-propylheptyl-i-decanoic acid ester, 2-propylheptyl-n-undecanoic acid ester, 2-propylheptyl-i-undecanoic acid ester, 2-propylheptyl-n-undecenoic acid ester, 2-propylheptyl-i-undecenoic acid ester, 2-propylheptyl-n-dodecanoic acid ester, and 2-propylheptyl-i-dodecanoic acid ester are the preferred reaction products of 2-propylheptanol using the transesterification process of the invention.

### Catalysts and other additives

**Lithiumhydroxide** is preferably used as monohydrate (LiOH*H₂O). The monohydrate is available in solid powder form and is thus easily to handle depending on the equipment of the reaction plant especially when water should be avoided during the reaction. It is therefore possible to add a powder of lithium hydroxide to the reactants and keep the amount of water in the reactor at a minimum in order to avoid further drying steps.

On the other hand lithiumhydroxide monohydrate has a good solubility in water and can also be added as an aqueous solution depending on the reaction plant and capabilities for charging of raw materials to the reaction vessel. Adding lithium monohydroxide in form of an aqueous solution avoids the development of dangerous dusts of lithium monohydroxide if necessary drying steps are acceptable.

It has been shown that using an aqueous solution of 5 to 13 wt%, preferably 8 to 12 wt %, more preferably 10 wt % ±1 wt % lithium hydroxide monohydrate based on the weight of the aqueous solution of lithium hydroxide monohydrate did not render the process inefficient when the "reaction mixture" is dried before transesterification.

The amount of lithiumhydroxide is depending on the used alcohol. For the transesterification with a monofunctional alcohol 0.01 to 0.001 mol lithium hydroxide per mol of the monofunctional alcohol b1 are used. As lithiumhydroxide is the reacting agent this amount is calculated for the lithium hydroxide and not for the respective hydrate, preferably 0.008 to 0.002 mol lithium hydroxide per mol of the monofunctional alcohol b1, more preferably 0.005 to 0.003 mol lithium hydroxide per mol of the monofunctional alcohol b1 are used.

For the transesterification with a polyfunctional alcohol 0.08 to 0.001 mol lithiumhydroxide per mol of the polyfunctional alcohol b1, preferably 0.04 to 0.001 mol lithiumhydroxide per mol of the polyfunctional alcohol b1 more preferably 0.02 to 0.005 mol lithiumhydroxide per mol of the polyfunctional alcohol b1 are used.

**Magnesiumoxide** is added to the reactants in an amount of 0.02 to 0.001 mol of magnesiumoxide per mol of the alkylester, preferably 0.007 to 0.003 mol of magnesiumoxide per mol of the alkylester, more preferably 0.006 to 0.004 mol of magnesiumoxide per mol of the alkylester in order to achieve the optimized transesterification speed.

From the prior art it was known that calciumoxide is better dissolved in organic environment and an effective catalyst. It was expected that the use of magnesiumoxide was disadvantageous in view of its catalytic activity due to its low solubility. Surprisingly magnesiumoxide works well in combination with lithium hydroxide although the dissolution of magnesiumoxide in organic environment is low. In addition it resulted in a more stable transesterified reaction product, not developing any precipitation during storage.

Apart from the catalysts optionally **sodium hypophoshite** could be added to the reactants to improve the product quality. In a preferred embodiment of the invention the process is conducted in the presence of sodium hypophosphite monohydrate in order to improve the color of the resulting reaction product and achieve a better peroxide number.

Sodium hypophosphite monohydrate is added in an amount of 0.01 to 0.3 wt % based on the sum of the weights of all educts without catalysts (which is the sum of the weight of reactants a) alkyl ester of a carboxylic acid and b) alcohol including further added raw materials such as an inert constituent if added), preferably sodium hypophosphite monohydrate is added in an amount of 0.02 to 0.2 wt % based on the sum of the weights of all educts without catalysts, more preferably in an amount of 0.04 to 0.1 wt % based on the sum of the weights of all educts without catalysts and most preferably in an amount of 0.04 to 0.06 wt % based on the sum of the weights of all educts without catalysts.

Hence preferably the transesterification process is conducted in the presence of sodium hypophosphite monohydrate (NaH₂PO₂* H2O) and the catalysts lithiumhydroxide monohydrate (LiOH*H₂O) and magnesiumoxide (MgO).

Another object of the invention is a catalyst composition comprising two single catalysts selected from lithiumhydroxide and magnesiumoxide (MgO), preferably selected from lithium hydroxide monohydrate and magnesiumoxide (MgO). It is relevant that both catalysts are used as physically separate components, so that a doping of the earth alkali - magnesium oxide - with the strong base lithiumhydroxide is excluded as this composition will not catalyse the transesterification process properly.

For base catalysed reactions MgO is often doped with alkali metal cations, in particular lithium hydroxide before adding this catalyst as a single catalyst to the reaction mixture, this doting process affords a preparation which is not achieved by simple mixing of lithiumhydroxide with magnesiumoxide.

For the current inventive transesterification stronger basic active sites are needed, so that doping MgO with lithium ions is to be avoided and explicitly excluded for the inventive process.

The preferred catalyst composition comprises lithium hydroxide and magnesiumoxide (MgO) in a molar ratio of from 1:3 to 3 :1, more preferably 1 : 2 to 2:1.

### Reaction conditions

In general after drying the raw materials in the reactor the alkyl ester a) and the alcohol b) are transesterified with the catalysts at increasing temperature up to 190 ° C first at atmospheric pressure and later under vacuum. During the reaction, the alcohol formed is continuously removed. At the end of the reaction, the excess alkyl ester is distilled off.

The transesterification is carried out at temperatures of 70°C to 240°C preferably at temperatures below 195°C, specifically 70 °C to 195 °C in order to reduce energy costs and to avoid exposure of the product to temperature stress, the most preferred range is 70 °C to 190 °C.

In a preferred embodiment, the process is carried out in the absence of solvents, preferably with educts which are substantially water-free. The respective catalysts can be used in a water-free form or in form of their hydrates. In case that they are added to the educts in an aqueous solution this mixture is dried before the transesterification reaction is started.

In another preferred embodiment the transesterification takes place in the present of an inert constituent, which is characterized in that it is not transesterified and is an oil of a natural source. Preferably the inert constituent is selected from the group consisting of elaeis guiineensis oil, passiflora incarnata seed oil, olive oil, olus oil, butyrospermum parkii butter, cocos nucifera oil, shorea stenoptera seed butter, almond oil, avocado oil, borage oil, canola oil, castor oil, chamomile, coconut oil, corn oil, cottonseed oil, jojoba oil, evening primrose oil, papaya oil, palm oil, hazelnut oil, peanut oil, walnut oil, safflower oil, sesame oil, soybean oil, sunflower oil, sweet almond, a rice bran/wheat germ oil, rosehip oil, ricinus communis oil, macauba oil, andiroba oil, Euphorbia vegetable oil and mixtures thereof. More preferably the inert constituent is selected from the group consisting of olive oil, almond oil, avocado oil, borage oil, canola oil, castor oil, coconut oil, corn oil, cottonseed oil, jojoba oil, palm oil, safflower oil, sesame oil, soybean oil, sunflower oil, macauba oil and mixtures thereof. Most preferably the inert constituent is selected from the group consisting of coconut oil and/or sunflower oil and/or macauba oil.

The inert constituent improves the mixing of components a) (alkyl ester) and b) (alcohol) resulting in an improved emulsification or in a dissolution of at least one of the components: It is not removed during or after the transesterification, so that it will be part of the final esterification product.

A preferred object of the invention is a transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms with a monofunctional alcohol (b1) of formula 2,

   R⁴-OH (formula 2),
wherein R⁴ is a linear or branched, saturated or unsaturated alkyl
in the presence of the catalysts lithiumhydroxide and magnesiumoxide (MgO) and optionally sodium hypophosphite.

Another preferred object of the invention is a transesterification process comprising treating an alkyl ester of a carboxylic acid (a) of formula 1b)

H₃C-(O)C - O-R³-O-C(O)-CH₃ (formula 1b)

wherein R³ is a saturated or unsaturated, branched or linear aliphatic residue with C4 to C18 carbon atoms with a monofunctional alcohol (b1) of formula 2,

   R⁴-OH (formula 2),
wherein R⁴ is a linear or branched, saturated or unsaturated alkyl
in the presence of the catalysts lithiumhydroxide and magnesiumoxide (MgO) and optionally sodium hypophosphite.

More preferred is the transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms with propylheptanol
in the presence of the catalysts lithiumhydroxide and magnesiumoxide (MgO) and optionally sodium hypophosphite.

Specifically preferred is a transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms with a propylheptanol in the presence of the catalysts lithiumhydroxide and magnesiumoxide (MgO) and sodium hypophosphite.

Another preferred object of the invention is a transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms
with a polyfunctional alcohol (b2) selected from glycerol, 1, 3-propylenglycol, 1,2-propylenglycol, ethylenglycol, 1,2-butandiol, 1,4-butandiol, 2,3-butandiol
in the presence of the catalysts lithiumhydroxide and magnesiumoxide (MgO) and optionally sodium hypophosphite.

Another preferred object of the invention is a transesterification process comprising treating an alkyl ester of a carboxylic acid (a) of formula 1b)

H₃C-(O)C - O-R³-O-C(O)-CH₃ (formula 1b)

wherein R³ is a saturated or unsaturated, branched or linear aliphatic residue with C4 to C18 carbon atoms with a polyfunctional alcohol (b2) selected from glycerol, 1, 3-propylenglycol, 1,2-propylenglycol, ethylenglycol, 1,2-butandiol, 1,4-butandiol, 2,3-butandiol
in the presence of the catalysts lithium hydroxide and magnesiumoxide (MgO) and optionally sodium hypophosphite.

More preferred is a transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms with glycerol in the presence of the catalysts lithiumhydroxide and magnesiumoxide (MgO) and optionally sodium hypophosphite.

Also preferred is a transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms with glycerol in the presence of a natural fatty oil and in the presence of the catalysts lithiumhydroxide and magnesiumoxide (MgO) and optionally sodium hypophosphite.

Specifically preferred is a transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms with glycerol in the presence of the catalysts lithiumhydroxide and magnesiumoxide (MgO) and sodium hypophosphite.

Also specifically preferred is a transesterification process comprising treating an alkyl ester of a carboxylic acid (a) of formula 1a)

R¹-C(O) O-CH₃ (formula 1a),

wherein R¹ is a saturated or unsaturated, branched or linear aliphatic residue with C4 to C18 carbon atoms with glycerol in the presence of a natural fatty oil and in the presence of the catalysts lithiumhydroxide and magnesiumoxide (MgO) and sodium hypophosphite.

The crude reaction product has a basic pH and must be neutralized for stability reasons and use in personal care or cosmetic compositions.

The transesterification process is preferably characterized in that the crude alkaline reaction mixture is neutralized with phosphoric or phosphorous acid (H₃PO₄, H₃PO₃) preferably with phosphoric acid up to a pH of 6.8 ±0.2. With phosphoric or phosphorous acid (H₃PO₄, H₃PO₃) dissolved lithium and magnesium cations are transferred into the respective phosphor salts, which are insoluble salts in the organic reaction mixture and can then easier be separated from the reaction product by filtration.

Further processing of the resulting ester is simplified as the effort to remove the catalysts as salts of the phosphoric acid is much lower. The salts can already be excellently separated during filtration, so that a simple filtration step may be sufficient to purify the product.

By simple filtration it was possible to almost completely remove the catalyst, so that also reaction products which cannot be distilled are sufficiently free of catalysts residues after filtration only.

### Examples

### Example 1 - Manufacturing of glycerolester in coconut oil

### Example 1 a - with lithiumhydroxide in aqueous solution

Production raw materials for example 1a:

| **Raw material** | **amount** |
|---|---|
| | |
| Coconut Oil, raff. (Charles Daudruy van Cauwenberghe) | 6735 kg |
| Methylester C8-C10 M (BASF) | 7410 kg |
| Glycerol 99.5% (BASF) | 1268 kg |
| Lithiumhydroxide solution 10% Lithiumhydroxide - Monohydrate powder (LiOH x 1H₂O) in water (Rockwood Lithium GmbH) | 40 kg |
| Magnesiumoxid (Luvomag M072 - Lehmann & Voss & Co.) | 10 kg |
| Sodiumhypophosphit monohydrate (Brenntag International) | 8 kg |

The coconut oil was filled to the reaction vessel at atmospheric pressure and heated up to 75°C. Methyl ester was added and subsequently 10 wt% lithium hydroxide solution (LiOH) was pumped into the reactor.

The mixture was dried at 50 mbar or at full pump vacuum and 70-85°C for 30 min. At a water content below 1000 ppm glycerol was filled into the reaction vessel.

Magnesium oxide (MgO) and sodium hypophosphite were added via a catalock.

After heating up to 190 °C the reaction took place at atmospheric pressure by distillation of methanol.

When methanol accumulation stagnated the vacuum ramp was started (ramp start value = normal pressure, ramp time = 3h, full scale = 350mbar)

When more than 2000 kg of methanol accumulated, distillate production was reduced, OHZ (hydroxyl value) and a SZ (acid number) were determined every 60 minutes from the vacuum start. Methyl ester (component a1) distillation was started at an OHZ of 50.

Methylester distillation and filtration of the product:
Methylester distillation was started with a second vacuum ramp (ramp start value = 300 mbar, full scale = 0 mbar (best vacuum), ramp time = 1.0 hours) at 150 to 190°C.

After expiry of the vacuum ramp, distilling at full vacuum was continued until no more methyl ester was produced. Then the vacuum pump was switched off and the vacuum extinguished with nitrogen. Color and OHZ were determined.

The reaction mixture was cooled to 60-75°C and pumped to the refining vessel. Phosphoric acid (aqueous solution 10%) was added at 60-75°C while stirring and the pH was determined to reach pH 6.9 ±0.2.

The batch was then filtered with activated coal and filter aids (Dicalite Speedplus, (Source?)) and bleaching earth (Tonsil Standard 310 FF).

The yield of the final product (after filtration) was 12500 kg with a density of 0.909kg/l Subsequently this transesterification ester was desodorized with steam in a counter current column.

### Example 1 b - with lithium hydroxide monohydrate in powder form

Production raw materials for example 1b:

| **Raw material** | **amount** |
|---|---|
| | |
| Coconut Oil, raff. (Charles Daudruy van Cauwenberghe) | 7090 kg |
| Methylester C8-C10M (BASF) | 7800 kg |
| Glycerol 99,5% (BASF) | 1335 kg |
| Lithiumhydroxide monohydrate (Rockwood Lithium GmbH) | 3.25 kg |
| Magnesiumoxid (Luvomag M072 - Lehmann & Voss & Co.) | 9.74 kg |
| Sodiumhypophosphit monohydrate (Brenntag International) | 16.23 kg |

The reaction of example 1a was repeated by adding solid lithium hydroxide monohydrate to the reaction mixture:
C8-C10 -methyl ester, coconut oil (preheated to 70°C) and glycerol (99.5 wt%) were added to the reaction vessel and dried at 70°C and vacuum up to a water content below 300 ppm.

Then lithium hydroxide monohydrate, magnesium oxide and NaH₂PO₂ monohydrate were added in powder form. The transesterification was started at athmosheric pressure increasing the temperature from 70° to 190°C with distillation of released methanol.

The product was further treated according to example 1a.

### Example 2 - Manufacturing of 2-propylheptanoloctylester

### Example 2 a - with lithiumhydroxide in 10 wt % aqueous solution

Production raw materials for example 2a:

| **Raw material** | **amount** |
|---|---|
| Agnique ME 890-GTTS (95 % C8) (BASF) | 7840 kg |
| 2-PROPYLHEPTANOL (BASF) | 6870 kg |
| Lithiumhydroxide solution 10% (Lithiumhydroxidemonohydrate powder (LiOH x 1H₂O) in water) (Rockwood Lithium GmbH) | 40 kg |
| Magnesiumoxid (Luvomag M072 - Lehmann & Voss & Co.) | 10 kg |
| Sodiumhypophosphit monohydrate (Brenntag International) | 8 kg |

The reaction vessel was filled with 2-propylheptanol, methyl ester and lithium hydroxide solution (LiOH) and dried at 50 mbar and 70-85°C up to a target value of less than 1000 ppm water (< 0.1 wt%). The vacuum was released, magnesium oxide (MgO) and sodium hypophosphite monohydrate were then added via the catalock.

Subsequently the reaction was started at atmospheric pressure by heating the reactor from 70 °C up to 190°C (dephlegmator 50°C) with the distillation of released methanol.

When methanol accumulation was diminished a vacuum ramp was started (ramp start value = normal pressure, ramp time = 3h, ramp end value = 350mbar). OHZ (hydroxyl value) and a SZ (acid number) were determined every 60 minutes from the vacuum start.

After reaching the full vacuum ramp value, a GC-analysis was conducted every hour, the reaction was continued until the methyl ester and alcohol content no longer decreased and the hydroxyl value was below 55 mg KOH/g. Then heating was stopped so that the temperature fell to 85°C and the methyl ester distillation was started at an OHZ of 50 mg KOH/g.

Raffination:
When the temperature dropped to 65 °C an aqueous solution of 10 wt% phosphoric acid (phosphoric acid 85% diluted with dem. water up to 10 wt% aqueous solution) was added to the reaction mixture and stirred for one hour to result in a pH below 7 (6.8 ±0.2).

The resulting neutralized product was dried (at 70 to 85°C and vacuum). After reaching a water residue below 0.10% (1000 ppm) the mixture is cooled to 50°C and filtered using diatomaceous earth and Tonsil Optimum (Clariant) as filtration aids.

The resulting yield was 12.555 kg 2-propylheptanoloctylester with a density of 0.855.

Fractionation and Desodoration:
The resulting filtrated transesterification product was fractionated, removing unreacted 2-propylheptanol and methylester as distillate in a first step and distilling the transesterification product in a second step.

Subsequently this ester was desodorized with steam in a counter current column.

### Example 2 b - with lithiumhydroxide monohydrate in powder form

Production raw materials for example 2b:

| **Raw material** | **amount** |
|---|---|
| Agnique ME 890-GTTS (95 % C8) (BASF) | 8250 kg |
| 2-PROPYLHEPTANOL (BASF) | 7100 kg |
| Lithiumhydroxide monohydrate (Rockwood Lithium GmbH) | 3.84 kg |
| Magnesiumoxid (Luvomag M072 - Lehmann & Voss & Co.). | 9.21 kg |
| Sodiumhypophosphit monohydrate (Brenntag International) | 7.68 kg |

The production of a 2-propylheptyl octanoic acid ester of example 2 a was repeated by adding solid lithium hydroxide monohydrate instead of an aqueous solution to the reaction mixture:
2-propylheptanol, octanoic acid methyl ester were added into the reaction vessel. The mixture was dried at 70°C and vacuum for approximately 30 minutes up to a water content below 300 ppm. Then lithium hydroxide monohydrate, magnesium oxide and NaH₂PO₂ monohydrate were added and the mixture was heated under nitrogen from 70°C up to 190° C with distillation of released methanol.

When methanol accumulation was diminished a vacuum ramp was started (ramp start value = normal pressure, ramp time = 3h, ramp end value = 350mbar). OHZ (hydroxyl value) and a SZ (acid number) were determined every 60 minutes from the vacuum start.

After reaching the full vacuum ramp value, a GC-analysis was conducted every hour, the reaction was continued until the methyl ester and alcohol content no longer decreased and the hydroxyl value was below 55 mg KOH/g. Then heating was stopped so that the temperature fell to 85°C and the methyl ester distillation was started at an OHZ of 50 mg KOH/g.

Raffination:
When the temperature dropped to 65 °C an aqueous solution of 10 wt% phosphoric acid (phosphoric acid 85% diluted with dem. water up to 10 wt% aqueous solution) was added to the reaction mixture and stirred for one hour to result in a pH below 7 (6.8 ±0.2).

The resulting neutralized product was dried (at 70 to 85°C and vacuum). After reaching a water residue below 0.10% (1000 ppm) the mixture is cooled to 50°C and filtered using diatomaceous earth and Tonsil Optimum (Clariant) as filtration aids.

The resulting yield was 12.555 kg 2-propylheptanoloctylester with a density of 0.855.

Fractionation and Desodoration:
The resulting filtrated transesterification product was fractionated, removing unreacted 2-propylheptanol and methylester as distillate in a first step and distilling the transesterification product in a second step.

Subsequently this ester was desodorized with steam in a counter current column.

### Example 3: Storage Stability, Residual Catalyst and Evaluation

Analytical parameters during and after manufacturing and before and after storage have been determined according to the following methods:
Acid number: determined according to ISO 4314
Peroxide number: determined according to ISO 3960
Saponification number: determined according to ISO 3681
Hydroxyl number: determined according to DIN 53240
Hazen Color: determined according to DIN ISO 6271

### 3.1 An 8-months storage test at 40°C was conducted with transesterification products manufactured with lithium hydroxide monohydrate powder and magnesiumoxide:

Cocoglycerides of example 1b:

| Specification | < 2.0 | < 3.0 | < 150 |
|---|---|---|---|
| Months at 40°C | Acid number | Peroxide number | Hazen Color (according to) |
| 1 | 0.35 | 1.28 | 100 |
| 3 | 0.37 | 0.73 | 108 |
| 8 | 0.30 | 0.3. | 129 |

2-Propylheptyl octanoic acid ester of example 2b:

| Specification | < 2.0 | < 1.0 | < 15 |
|---|---|---|---|
| Months at 40°C | Acid number | Peroxide number | Hazen Color |
| 1 | 0.06 | 0.02 | 3 |
| 3 | 0.10 | 0.09 | 2 |
| 6 | 0.02 | 0.02 | 7 |
| 8 | 0.06 | 0.02 | 1 |

### 3.2 Accelerated 4-weeks storage tests at 50 °C were conducted with transesterification products manufactured with lithium hydroxide monohydrate - 10 wt% aqueous solution and magnesiumoxide:

Cocoglycerides of example 1a:

| properties | units | specification | Batch 1 - before storage | Batch 2 - before storage | Batch 1 - after 4-weeks storage | Batch 2 - after 4-weeks storage |
|---|---|---|---|---|---|---|
| acid number | mg KOH/g | ≤ 2 | 0.35 | 0.29 | 0.34 | 0.24 |
| saponification value | mg KOH/g | ≥265 to ≤ 295 | 288 | 286 | 288 | 289 |
| peroxide number | meq | ≤ 3.0 | | | 1.0 | 0.2 |
| Hazen color | | ≤150 | 110 | 75 | 83 | 83 |

2-Propylheptyl octanoic acid ester of example 2a:

| properties | units | specification | Batch 1 - before storage | Batch 2 - before storage | Batch 1 - after 4-weeks storage | Batch 2 - after 4-weeks storage |
|---|---|---|---|---|---|---|
| acid number | mg KOH/g | ≤ 0.2 | 0.02 | 0.07 | 0.09 | 0.09 |
| saponification value | mg KOH/g | ≥190 to < 205 | 198 | 197 | 196 | 196 |
| peroxide number | meq | ≤ 1 | 0 | 0 | 0.1 | 0.1 |
| Hazen color | | ≤15 | 3 | 8 | 7 | 8 |

### 3.3 Determination of residual catalyst

Analytical determination of lithium, magnesium, sodium and total phosphorus (according to DIN EN ISO 11885, Fa. Fülling) in the transesterification products of examples 1a, b and 2a, b after filtration resulted in values below quantification level for all ions and all examples.

### 3.4 Evaluation of the results

It could be verified that the use of lithiumhydroxide and magnesiumoxide as catalyst mixture simplifies the manufacturing process of esters manufactured by transesterification. The process of the invention results in esters of sufficient purity and stability avoiding further washing steps of the transesterified product which would have been necessary in case of the use of organotin based catalysts. A complex precipitation and purification of organotin or titanate catalysts with additional bleaching steps of the reaction product could be avoided, so that the production is much more energy and resource saving as commonly used transesterification with organotin or titanate catalysts. The catalyst comprising litiumhydroxide monohydrate and magnesiumoxide could easily be removed, so that no residual catalyst could be detected in the transesterified reaction product.

## Claims

1. A transesterification process comprising
treating an alkyl ester of a carboxylic acid (a) of formula 1a or 1b
R¹-C(O) O-R² (formula 1a)
R²-(O)C - O-R³-O-C(O)-R² (formula 1b)
wherein R¹ and R³ is a saturated or unsaturated, branched or linear aliphatic or aromatic residue and
R² is selected from the group consisting of methyl, ethyl, propyl, isopropyl, n-butyl, and 2,2-butyl,
with a monofunctional alcohol (b1) of formula 2,
R⁴-OH (formula 2),
wherein R⁴ is a linear or branched, saturated or unsaturated or alkoxylated alkyl-, alkenyl- or alkoxy residue or
a polyfunctional alcohol (b2) selected from glycerol, 1, 3-propylenglycol, 1,2-propylenglycol, ethylenglycol, 1,2-butandiol, 1,4-butandiol, 2,3-butandiol
in the presence of the catalysts lithiumhydroxide and magnesiumoxide (MgO) and optionally sodium hypophosphite.

2. The transesterification process according to claim 1 wherein the moiety R¹ in formula 1a is a linear, branched, saturated or unsaturated C1 -C 36 alkyl and R³ in formula 1b is a linear, branched, saturated or unsaturated C 2 -C 54 alkyl.

3. The transesterification process according to claim 1 and/or claim 2, wherein the monofunctional alcohol (b1) is 2-propylheptanol and/or the polyfunctional alcohol (b2) is glycerol.

4. The transesterification process according to any one of claims 1 to 3, wherein the moiety R¹ in formula 1a is a linear, branched, saturated or unsaturated C 4 -C 18 alkyl.

5. The transesterification process according to any one of claims 1 to 4 wherein the catalysts comprise lithiumhydroxide monohydrate (LiOH*H₂O) and magnesiumoxide (MgO) and optionally sodium hypophosphite monohydrate.

6. The transesterification process according to any one of claims 1 to 5 in the presence of sodium hypophosphite monohydrate (NaH₂PO₂* H2O) and the catalysts lithiumhydroxide monohydrate (LiOH*H₂O) and magnesiumoxide (MgO)

7. The transesterification process according to any of claims 1 to 6, **characterized in that** it is conducted at temperatures of 70°C to 240°C preferably 70 °C to 195 °C.

8. The transesterification process according to any of claims 1 to 7, **characterized in that** 0.01 to 0.001 mol lithiumhydroxide per mol of the monofunctional alcohol (b1) are used.

9. The transesterification process according to any of claims 1 to 7, **characterized in that** 0.08 to 0.001 mol of lithiumhydroxide per mol of the polyfunctional alcohol (b2) are used.

10. The transesterification process according to any of claims 1 to 9, **characterized in that** 0.02 to 0.001 mol of magnesiumoxide per mol of the alkylester (a) are used.

11. The transesterification process according to any of claims 1 to 10, **characterized in that** 0.01 to 0.3 wt % sodium hypophosphite based on the sum of the weights of all reactants without catalysts are used.

12. The transesterification process according to any of claims 1 to 11, **characterized in that** the crude alkaline reaction mixture is neutralized with phosphoric acid.

13. The transesterification process according to any of claims 1 to 12, **characterized in that** it is conducted in the presence of an inert constituent, which is not transesterified and is an oil of a natural source.

14. A catalyst composition for use in a transesterification process, comprising a mixture of two single catalysts selected from lithiumhydroxide and magnesiumoxide (MgO).

15. The catalyst composition according to claim 12, comprising lithiumhydroxide and magnesiumoxide (MgO) in a molar ratio of from 1 : 3 to 3:1.
